# EUROPEAN PATENT SPECIFICATION

(11) **EP 0 403 243 B1**
(45) Date of publication and mention of the grant of the patent: **08.11.1995**
(21) Application number: 90306428.5
(22) Date of filing: 13.06.1990
(51) Int. Cl.: C07K 14/58, A61K 51/08

(54) **Chelate derivatives of atrial natriuretic factor (ANF)**
Chelat-Derivate von atrialnatriurethischem Faktor (ANF)
Dérivés chélateurs du facteur natriurétique Atrial (ANF)

(30) Priority: 16.06.1989 US 367169
(43) Date of publication of application: 19.12.1990
(73) Proprietor: MERCK FROSST CANADA INC., Kirkland Quebec H9H 3L1 (CA)
(72) Inventor: Flanagan, Richard J., Hudson, Quebec H9J 2EJ (CA); Charleson, F. Peter, Kirkland, Quebec H9J 2E9 (CA)
(74) Representative: Cole, William Gwyn

(56) References cited:
- EP-A- 0 315 188
- US-A- 4 609 725
- ACC. CHEM. RES., vol. 17, 1984, pages 202-209, Washington, US; MEARES et al.: "Metal chelates as probes of biological systems"
- NUCL. MED. BIOL., vol. 15, no. 6, 1988, pages 659-664, Oxford, GB; HUNT: "Potential radiopharmaceuticals for the diagnosis of biliary atresia and
- neonatal hepatitis: EHPG and HBED chelates of 67Ga and 111In"

## Description

### BACKGROUND OF THE INVENTION

It has been postulated for many years that the cardiac atria serve as sensors that are important in detecting changes in extracellular fluid volume (Gauer et al., Physiol, Rev. 43: 423, 1963). Such a receptor function for the cardiac atria is known in the case of vasopressin, the hypothalamic hormone important in regulating the osmotic concentration of the body fluids.

The postulated existance of a substance which would enhance urinary sodium excretion, and hence be involved in regulation of extracellular fluid volume, was demonstrated recently. De Bold et al., Life Sci. 28: 89, 1981, injected a partially purified extract of cardiac atria of rats into other anesthetized rats and observed a large increase in urine flow and in urinary sodium excretion. This relatively crude extract possessed the appropriate characteristics of an endogenous natriuretic substance.

In addition to its potent diuretic and natriuretic effects, properties that make the material especially appropriate to exert a major effect on body fluid volume regulation, it was also discovered that these extracts of cardiac atria have potent smooth muscle relaxant activity (Currie et al., Science 221: 71, 1983). Such action implies a potential direct role in regulating blood pressure as well as a role in regulating extracellular fluid volume.

Because of the immediately recognized importance of this discovery for understanding the regulation of body fluid volume and blood pressure and the obvious therapeutic potential of such a natural substance in the treatment of congestive heart failure and hypertension, numerous laboratories set about to isolate, characterize and chemically identify the active substance(s) in the cardiac atrial extracts. The active substance(s) in cardiac atria was called atrial natriuretic factor or ANF but has been referred to also as cardionatrin (de Bold et al., Life Sci. 33: 297-302, 1983) and atriopeptin (Currie et al., Science 111: 67, 1984). Atrial natriuretic factor was shown to be a family of peptides all of which have a common amino acid sequence but differ in length by the presence or absence of 1-8 amino acids on the amino or carboxyl termini.

Peptide chemists quickly produced completely synthetic material that mimicked the biological activity of the family of peptides that have been isolated from the cardiac atria.

The biological activity of ANF indicates utility in congestive heart failure where standard therapy utilizes potent diuretics in combination with peripheral vasodilating drugs. Atrial natriuretic factor combines both of these actions in one molecule which is produced naturally within the body. It is possible that the salt and water retention associated with congestive heart failure is a result of inadequeate production of ANF. If such is true, administration of ANF would allow for replacement of adequate quantities of the material.

A second major disease for which the biological activity of ANF indicates utility is essential hypertension. Standard therapy for hypertension utilizes diuretic and peripheral vasodilating drugs. Atrial natriuretic factor incorporates both of these characteristics. A specific use also may be found in the acute treatment of hypertensive crisis such as malignant hypertension where the powerful vasodilating effect of ANF would be paramount.

In addition to these two very broad categories of therapeutic utility, it is possible that those diseases which are characterized by decreases in renal function may benefit because of the favorable action of ANF on renal hemodynamics, especially enhancement of medullary blood flow.

U.S. Patent 4,609,725 by Brady et al. teaches that synthetic fragments of ANF can be labelled at carboxyl terminus in the tyrosine residue by electrophillic labelling with iodine and that these radiolabelled derivatives are useful for studying the in vivo and in vitro metabolism of ANF.

Radioactive indium complexes with EDTA-type chelate systems are described by Meares and Wensel in Acc. Chem. Res., 1984, 17, 202-209. EHPG and HBED chelates of gallium and indium are proposed as potential radiopharmaceuticals by F.C. Hunt in Nucl. Med. Biol., 1988, 15, 659-664. In neither of these publications, however, is there any disclosure or suggestion of ANF-type modifications of the proposed complexes.

### OBJECTS OF THE INVENTION

It is the object of the present invention to provide radioactive derivatives of synthetic fragments of ANF in which the radioactive atom is attached to the N-terminus of the peptide by means of a chelate molecule.

It is another object of the present invention to provide easier access to radiolabelled forms of ANF than are presently available by means of electrophilic labelling with iodine, since chelate chemistry allows the synthesis of the radioactive form of ANF by the mere mixing of the chelated form of ANF with a radioactive metal.

A further object of the invention is to provide a compound useful for studying the metabolic and half-life of ANF in vivo and in vitro.

These and other objects of the invention will be apparent from the following description.

### SUMMARY OF THE INVENTION

Radiolabelled forms of ANF may be produced by combining a synthetic derivative of mammalian atrial natriuretic factor which contains a chelate functionality at the N-terminal with a suitable metallic radioisotope such as Tc-99m, Ga-67 or In-111.

### DETAILED DESCRIPTION

According to the present invention the chelate form of ANF has the following amino acid sequence: 
A-Cys-Phe-Gly-Gly-Arg-X-Asp-Arg-Ile-Gly-Ala-Gln-Ser-
Gly-Leu-Gly-Cys-Asn-Ser-Phe-Arg-Tyr-B;
where X is Ile or Met;
and A is: 
Ch-Ser-
Ch-Ser-Ser-
Ch-Arg-Ser-Ser-
Ch-Arg-Arg-Ser-Ser-
Ch-Leu-Arg-Arg-Ser-Ser-
Ch-Ser-Leu-Arg-Arg-Ser-Ser-
Ch-Arg-Ser-Leu-Arg-Arg-Ser-Ser-
Ch-Pro-Arg-Ser-Leu-Arg-Arg-Ser-Ser-
Ch-Gly-Pro-Arg-Ser-Leu-Arg-Arg-Ser-Ser-
Ch-Ala-Gly-Pro-Arg-Ser-Leu-Arg-Arg-Ser-Ser-
Ch-Leu-Ala-Gly-Pro-Arg-Ser-Leu-Arg-Arg-Ser-Ser-
and B is absent or present and if present is:
Arg
Arg-Arg
and where Ch is:
a chelate residue selected from the group consisting of: 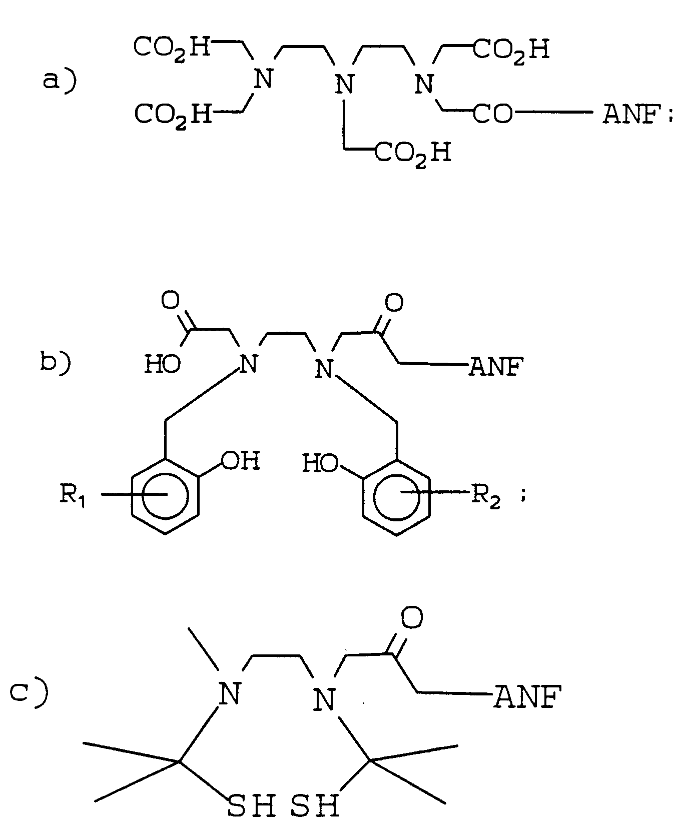 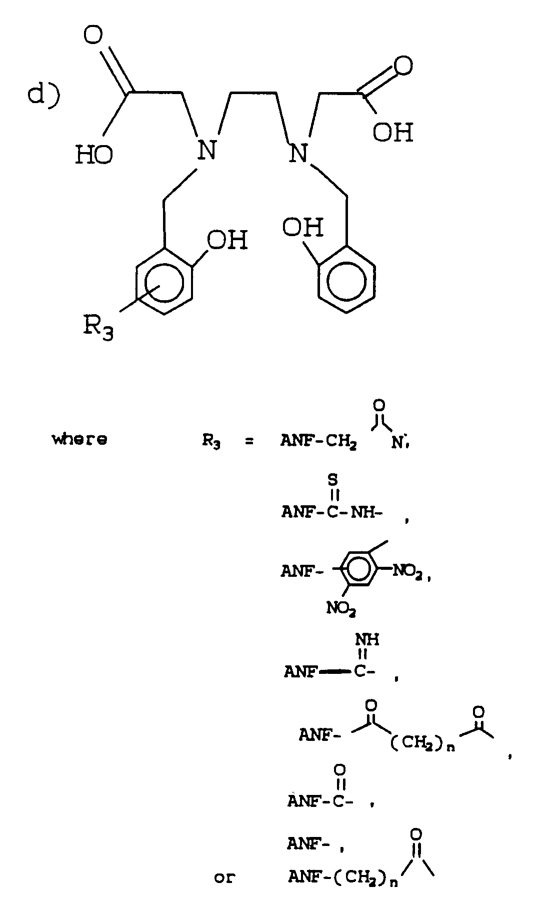
and where n is 2 to 6 and the peptide is linear or cyclized by means of covalent linkages between the two cysteine residues and R₁R₂ which may be the same or different, is selected from hydrogen, C₁-C₇ alkyl, C₁-C₇ alkoxy, C₃-C₇ cycloalkyl, amino, halo, i.e., chloro, bromo, fluoro and iodo, trifluormethyl and the like.

Preferably, X is Ile or Met.

In the above where X is Ile, A is a chelate as defined such as HBED-Arg-Arg-Ser-Ser and B is absent is a preferred chelate and HBED is N,N′-bis-[-2-hydroxybenzyl]ethylenediamine-N,N′-diacetic acid.

The chelated form of ANF is synthesized by first converting the ANF derivative to the tetra-hydrochloride form by passage through a suitable ion-exchange column. This is especially important if the ANF is originally in the tetraacetate form. The tetrahydrochloride form is then reacted with an active ester derivative of the chelate molecule in such a way that reaction occurs at the N-terminal of the peptide. The resulting mixture of chelated ANF, unchelated ANF and unreacted active chelate ester is then purified by preparative HPLC to obtain the pure chelated ANF derivative. This chelated ANF form is then reacted in ion-free water with a metallic radioisotope, such as ⁶⁷ Ga, gallium citrate, ¹¹¹ In, indium chloride or ^{99m}Tc-technetium ions in the IV or V state.

In practice these modified chelate forms of ANF are dissolved in a buffer carrier, lyophillized and delivered to the end user in unit doses of 10 to 300 micrograms of ANF per vial. The user will add a solution of a metallic radioisotope to the lyophillized chelate and after a period of minutes during which the metal chelate complex forms the labelled ANF complex is ready for use.

Radioisotopes for which these chelates are suitable are ^{99m}Technetium, ⁶⁷Gallium, ⁶⁸Gallium, ⁵¹Chromium, ⁵⁷Cobalt, ⁶⁰Cobalt, ¹¹¹Indium, ^{113m}Indium, ¹⁸⁶Rhenium, ¹⁸⁸Rhenium, ⁹⁰Yttrium and the like.

In order to further illustrate the practice of the present invention, the following Examples are included.

### EXAMPLE 1

### Preparation of ANF tetra-hydrochloride

Human ANF fragment (methionine at the 10 position) was examined by 300 MHz NMR spectrometry to confirm the presence of the the tetra-acetate groups. These groups are found at σ 1.94 in Fig. 1. 10 milligrams of this sample was dissolved in 2 mL of ultra-pure water (resistance ≧ 18 MOhms) and passed through a small column containing 1 gram of Dowex AG3-X4 (chloride form). The eluate was recovered and the column further washed with ultrapure water. The tetrahydrochloride ANF was recovered from the eluate by lyophilization. Yield 9.6 mg. The NMR spectrum of this material confirms the absence of acetate residues at σ 1.9 to σ 2.1 (Fig. 2)

### EXAMPLE 2

### Reaction of ANF with N,N-bis[2-(2,6-dioxomorpholino)-ethyl]-glycine.

1.0 mG of tetra-hydrochloride human ANF (methionine at the 10 position) was reacted in 40 microliters of 0.1M NaHCO₃ buffer at pH 8.2 with a solution of N,N-bis[2-(2,6-dioxomorpholino)-ethyl]-glycine (160 micrograms, 448 nmoles, in 4 microlitres of dimethyl sulphoxide (DMSO)). The mixture was allowed to stand overnight and then saturated with CO₂ gas. An analysis of this solution by means of radiochromatography (ITLC-silica gel/CO₂ saturated saline) was carried out with ¹¹¹-InCl₃ indicating that 82% of the radioactivity was bound to the ANF molecule.

This mixture of crude ANF derivative was purified by preparative HPLC chromatography, using a Waters C8 reverse phase column and 81:19 water/acetonitrile as solvent. The elution time of unchelated ANF using this system was 2.18 minutes and that of the chelated form was 3.28 minutes. The isolated yield of purified diethylenetriaminepentacetic acid ANF was 319 micrograms. The 300 MHz NMR spectrum of this material is shown in Fig. 3.

### EXAMPLE 3

### Reaction of the diethylenetriaminepentacetic acid derivative of ANF with ¹¹¹ In-Indium Chloride

25 microliter of ultrapure water was added to 25 microliters of ¹¹¹ Indium Chloride stock solution (Atomic Energy of Canada Limited). Add 100 microliters of 0.01M sodium citrate pH 5.0 buffer, followed by 10 microliters of solution containing 16 micrograms of the diethylenetriaminepent acetic acid derivative of ANF. Reaction is almost instantaneous and analysis by radiochromatography on ITLC indicated a labelling yield of 83%.

### EXAMPLE 4

### Preparation of N,N′-bis-[2-hydroxybenzyl] ethylenediamine-N,N′diacetic acid-N''-Hydroxy-Succinimide Ester.

106 mg of N,N′-bis[2-hydroxybenzyl]ethylenediamine-N,N′diacetic acid (HBED) was dissolved in 1 mL of acetonitrile and the solution saturated with trimethylamine gas. The insolubles were removed by filtration and the solute evaporated to dryness at 90° under high vacuum. The residue was dissolved in 5 mL of acetonitrile containing 205 mg of disuccinidimyl carbonate. After 1 hour the acetonitrile was removed under vacuum. The residue is quickly partitioned between water and chloroform and the chloroform layer is isolated, dried over sodium sulphate and removed in vacuo to give the crude ester. ¹HMR confirms the presence of two succinidimyl residues per HBED molecule. Since this is a very reactive ester it is used without further purification.

### EXAMPLE 5

### Reaction of ANF with N,N′-bis-[2-hydroxybenzyl]ethylenediamine-N,N′diacetic acid -N''-Hydroxy-Succinimide Ester.

0.38 mg of the above active ester was reacted with 1 mg of human atrial natriuretic factor tetrahydrochloride in 110 microlites of dry dimethyl sulphoxide and 2.7 microliter of dry pyridine. Analysis by thin layer chromatography showed the conversion of ANF (O-phthaldehyde positive, UV negative) to a new derivative (O-phthaldehyde negative, UV positive). After 16 hours the solution was evaporated to dryness and the residue taken up in methanol and the insoluble protein fraction isolated and purified by chromatography and lyophillized in lots of 0.5 mg.

### EXAMPLE 6

### Labelling of the N,N′-bis-[2-hydroxybenzyl]ethylenediamine-N,N′diacetic acid derivative of ANF with ¹¹¹ Indium Chloride.

2mCi of ¹¹¹InCl₃ in 1 mL of 0.1 M sodium citrate buffer (pH 5.4) was added to a vial containing 0.5 mg of lyophillized ANF. After 5 minutes the solution was analyzed by ITLC showing a labelling yield of 90%.

## Claims

1. A peptide containing one of the amino acid sequences: 
A-Cys-Phe-Gly-Gly-Arg-X-Asp-Arg-Ile-Gly-Ala-Gln-Ser-
Gly-Leu-Gly-Cys-Asn-Ser-Phe-Arg-Tyr-B
 where X is Ile or Met,
and A is: 
Ch-Ser-
Ch-Ser-Ser-
Ch-Arg-Ser-Ser-
Ch-Arg-Arg-Ser-Ser-
Ch-Leu-Arg-Arg-Ser-Ser-
Ch-Ser-Leu-Arg-Arg-Ser-Ser-
Ch-Arg-Ser-Leu-Arg-Arg-Ser-Ser-
Ch-Pro-Arg-Ser-Leu-Arg-Arg-Ser-Ser-
Ch-Gly-Pro-Arg-Ser-Leu-Arg-Arg-Ser-Ser-
Ch-Ala-Gly-Pro-Arg-Ser-Leu-Arg-Arg-Ser-Ser-
Ch-Leu-Ala-Gly-Pro-Arg-Ser-Leu-Arg-Arg-Ser-Ser-
 and B is absent or present and if present is:
Arg
Arg-Arg
and where Ch is:
a chelate selected from the group consisting of: 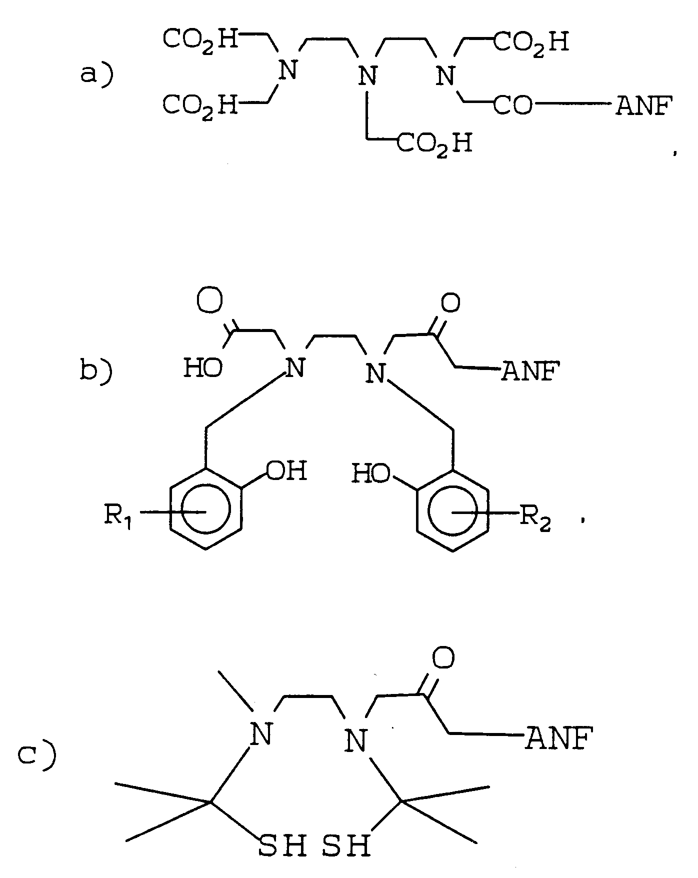 and 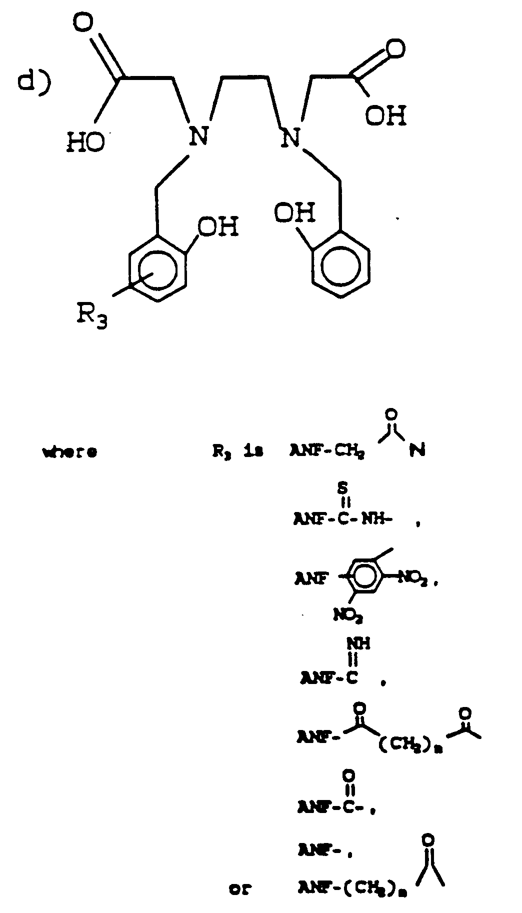 and wherein n = 2 to 6 and where the peptide is linear or cyclized by means of covalent linkages between the two cysteine residues, and R₁R₂ may be equal or different, is selected from, C₁-C₇ alkyl, C₁-C₇ alkoxy, C₃-C₇ cycloalkyl, halo, amino, and trifluoromethyl.

2. A peptide of Claim 1 where X is Ile.

3. A peptide of Claim 1 where X is Met.

4. A peptide of Claim 1 where X is Ile, A is HBED-Arg-Arg-Ser-Ser and B is absent.

5. A peptide according to claim 1 wherein the chelate is

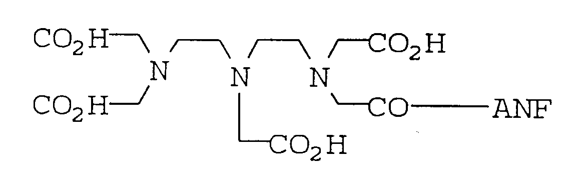

## Patentansprüche

1. Ein Peptid, enthaltend eine der Aminosäuresequenzen: 
A-Cys-Phe-Gly-Gly-Arg-X-Asp-Arg-Ile-Gly-Ala-Gln-Ser-Gly-Leu-
Gly-Cys-Asn-Ser-Phe-Arg-Tyr-B
 worin X Ile oder Met bedeutet,
A 
Ch-Ser-
Ch-Ser-Ser-
Ch-Arg-Ser-Ser-
Ch-Arg-Arg-Ser-Ser-
Ch-Leu-Arg-Arg-Ser-Ser-
Ch-Ser-Leu-Arg-Arg-Ser-Ser-
Ch-Arg-Ser-Leu-Arg-Arg-Ser-Ser-
Ch-Pro-Arg-Ser-Leu-Arg-Arg-Ser-Ser-
Ch-Gly-Pro-Arg-Ser-Leu-Arg-Arg-Ser-Ser-
Ch-Ala-Gly-Pro-Arg-Ser-Leu-Arg-Arg-Ser-Ser-
Ch-Leu-Ala-Gly-Pro-Arg-Ser-Leu-Arg-Arg-Ser-Ser-
 bedeutet
und B nicht vorhanden oder vorhanden ist, und wenn es vorhanden ist,
Arg
Arg-Arg bedeutet,
und worin Ch ein Chelat bedeutet, ausgewählt aus der Gruppe, die aus 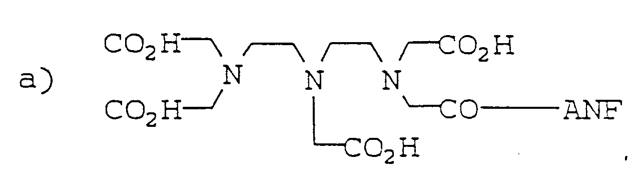 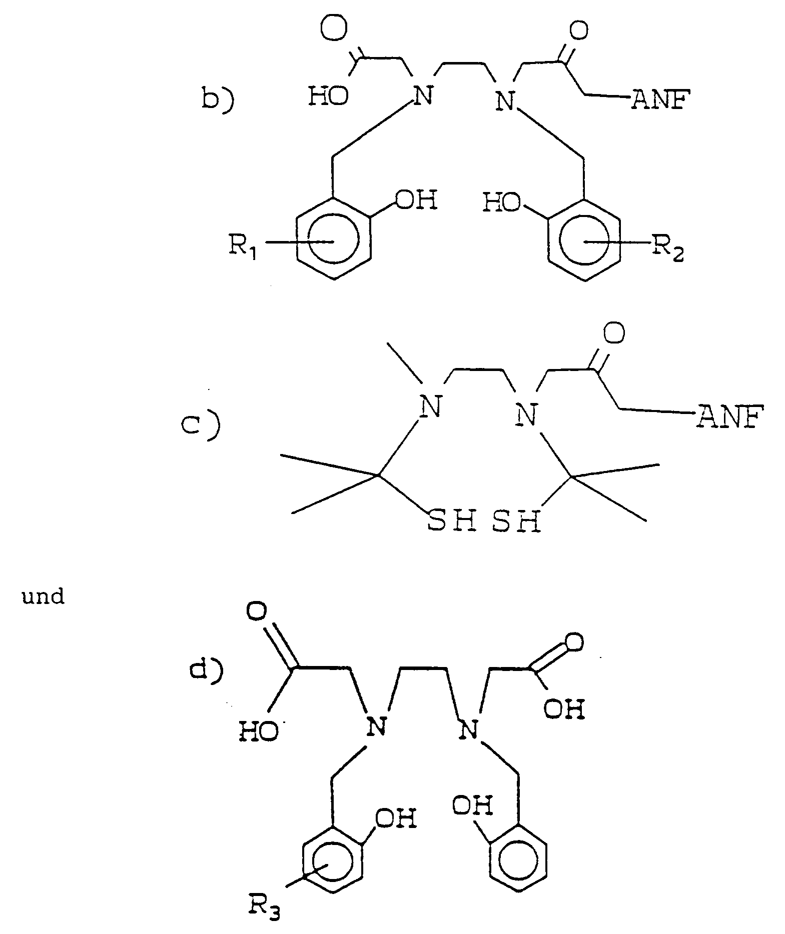 besteht, worin 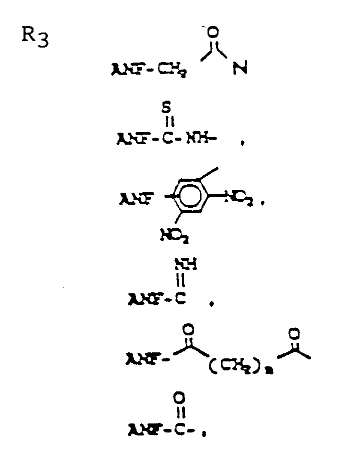 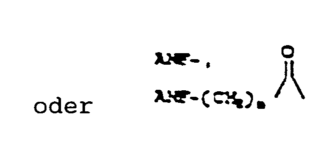 bedeutet,
und worin n 2 bis 6 ist,
und worin das Peptid linear oder mittels covalenter Bindungen zwischen den beiden Cysteinresten cyclisiert ist, und R₁R₂, die gleich oder verschieden sein können, aus C₁-C₇ Alkyl, C₁-C₇ Alkoxy, C₃-C₇ Cycloalkyl, Halogen, Amino und Trifluormethyl ausgewählt sind.

2. Ein Peptid des Anspruchs 1, worin X Ile ist.

3. Ein Peptid des Anspruchs 1, worin X Met ist.

4. Ein Peptid des Anspruchs 1, worin X Ile ist, A HBED-Arg-Arg-Ser-Ser ist und B nicht vorhanden ist.

5. Ein Peptid gemäß Anspruch 1, worin das Chelat 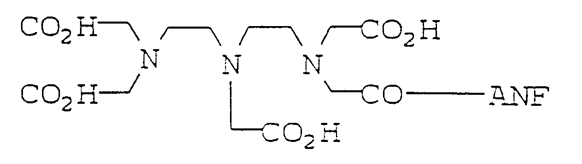 ist.

## Revendications

1. Peptide contenant la séquence d'aminoacides suivante : 
A-Cys-Phe-Gly-Gly-Arg-X-Asp-Arg-ile-Gly-Ala-Gln-Ser-
Gly-leu-Gly-Cys-Asn-Ser-Phe-Arg-Tyr-B ;
 dans laquelle X représente Ile ou Met,
et A représente : 
Ch-Ser-
Ch-Ser-Ser-
Ch-Arg-Ser-Ser-
Ch-Arg-Arg-Ser-Ser-
Ch-Leu-Arg-Arg-Ser-Ser-
Ch-Ser-Leu-Arg-Arg-Ser-Ser-
Ch-Arg-Ser-Leu-Arg-Arg-Ser-Ser-
Ch-Pro-Arg-Ser-Leu-Arg-Arg-Ser-Ser-
Ch-Gly-Pro-Arg-Ser-Leu-Arg-Arg-Ser-Ser-
Ch-Ala-Gly-Pro-Arg-Ser-Leu-Arg-Arg-Ser-Ser-
Ch-Leu-Ala-Gly-Pro-Arg-Ser-leu-Arg-Arg-Ser-Ser-
 et B est absent ou présent, et s'il est présent, il représente :
Arg
Arg-Arg
et Ch représente :
un groupe chélatant choisi parmi : 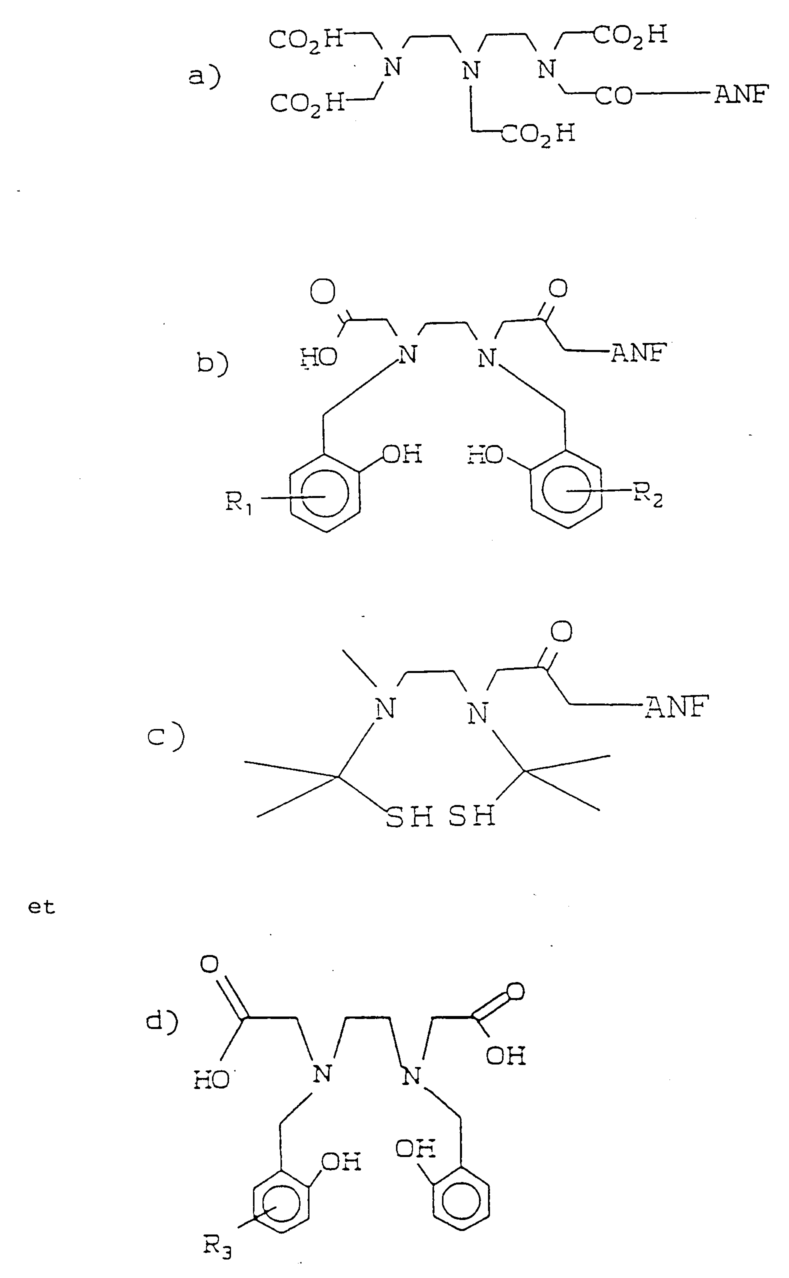 où 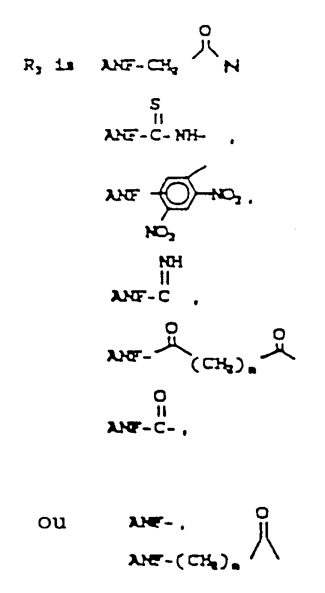 et n varie de 2 à 6,
et le peptide est linéaire ou cyclisé par l'intermédiaire de liaisons covalentes entre les deux résidus cystéine, et R₁ ainsi que R₂ peuvent être identiques ou différents, et ils sont choisis parmi les groupes alkyle en C₁-C₇, alkoxy en C₁-C₇, cycloalkyle en C₃-C₇, halo, amino et trifluorométhyle.

2. Peptide selon la revendication 1, dans lequel X représente Ile.

3. Peptide selon la revendication 1, dans lequel X représente Met.

4. Peptide selon la revendication 1, dans lequel X représente Ile, A représente HBED-Arg-Arg-Ser-Ser, et B est absent.

5. Peptide selon la revendication 1, dans lequel l'agent chélatant est :
